# EUROPEAN PATENT SPECIFICATION

(11) **EP 4 615 421 B1**
(45) Date of publication and mention of the grant of the patent: **02.09.2026**
(21) Application number: 23800861.9
(22) Date of filing: 06.11.2023
(51) Int. Cl.: A61K 9/16, A61K 9/20, A61K 31/55

(54) **GALANTAMINE MINITABLETS**
GALANTAMIN-MINITABLETTEN
MINI-COMPRIMÉS DE GALANTAMINE

(30) Priority: 07.11.2022 EP 22205887
(43) Date of publication of application: 17.09.2025
(73) Proprietor: Rejuvenate Biomed NV, 3590 Diepenbeek (BE)
(72) Inventor: BELIËN, Ann, 3590 Diepenbeek (BE); CORVELEYN, Sam, 3590 Diepenbeek (BE); VERHOEVEN, Ellen, 9052 Gent (BE)
(74) Representative: Arnold & Siedsma
(86) International application number: PCT/EP2023/080852
(87) International publication number: WO 2024/099974

(56) References cited:
- WO-A2-2008/062426
- US-A1- 2007 092 568
- US-A1- 2008 254 131
- US-A1- 2012 288 560

## Description

### FIELD OF THE INVENTION

The present invention relates to a method for obtaining galantamine minitablets, the minitablets obtainable by the method, and the use of these minitablets as a mono-therapeutic, or in combination with other active pharmaceuticals.

### BACKGROUND TO THE INVENTION

Aging is the gradual loss of function and deterioration at the cellular, tissue, and organ level, leading to a progressive loss of physiological integrity, an increased susceptibility to disease and external stressors, and eventually leading to death. As the global population aging is increasing, the incidences of age-related diseases are expanding every year. And hence, numerous attempts have been made in trying to treat the age-related diseases, as well as trying to delay the onset of the complex process of aging. As a result, a number of age-related pathways have been identified that might be targeted to extend life span and health span. For example, there is overwhelming evidence that single gene mutations in nutrient-sensing pathways, such as insulin/insulin-like growth factor (IGF) signalling or the mechanistic target of rapamycin (mTOR) signalling pathways, extend life span and health span in invertebrates. These pathways have also been evaluated in mammalian models, in which health span and life span have been extended by genetic manipulation or drugs. Although this raises hope for new interventions, including drugs that slow the aging process and slow the appearance of age-related disease by modulating conserved pathways of aging, so far, except for some symptomatic treatment, unfortunately, there is no known intervention that was shown to efficiently slow down the human aging process. After all, in addition to treating existing diseases and disorders by means of medicine, the necessity and demand for measures for staying healthy and delaying aging is increasing.

Metformin has widely been used and is approved as an anti-diabetic drug for the treatment of type 2 diabetes. It increases insulin sensitivity, and thereby improves insulin action at the cellular level without affecting insulin secretion. It has also been shown that metformin exerts positive effects on several cardiovascular risk factors. Furthermore, it has been shown that metformin targets a number of aging mechanisms as well. Specifically for aging, metformin leads to decreased insulin levels, decreased IGF-1 signalling, inhibition of mTOR, inhibition of mitochondrial complex I in the electron transport chain and reduction of endogenous production of reactive oxygen species, activation of AMP-activated kinase (AMPK), and reduction in DNA damage. Metformin was also shown to favourably influence metabolic and cellular processes closely associated with the development of age-related conditions, such as inflammation, autophagy, and cellular senescence (Barzilai et al., Cell Metab. 2016). Using a *C. elegans* model system, the health-promoting and life-prolonging effects of metformin in type 2 diabetes were confirmed as well. Human studies have further shown that metformin significantly reduces the risk of cancer in diabetic patients (Fuming et al. Oncol Lett. 2018) and lowers the risk for coronary disease (Hong et al., Diabetes Care. 2014). However, all these effects have been observed when administering metformin at a considerably high therapeutic dose, which is at least 850 mg/day or more. In addition, so far, no synergistic effects of metformin in combination with another compound on age-related diseases were identified.

Galantamine, an acetylcholinesterase inhibitor that allosterically modulates nicotinic receptors, is widely known as a drug administered to patients with Alzheimer's disease. In *C*. *elegans,* galantamine was shown to facilitate cholinergic neurotransmission in a similar manner as in humans, and to rescue the paralysis phenotype in a transgenic *C. elegans* Alzheimer's disease model (Xin et al., Plos One, 2013), but no effects on locomotion, mobility or other forms of age-related decline have been described for galantamine in *C. elegans.* In humans, it has been shown that galantamine significantly reduces death by myocardial infarction (Nordström et al., 2013). Furthermore, galantamine alleviates inflammation and insulin resistance in metabolic syndrome subjects (Consolim-Colombo et al.; JCI Insight. 2017). However, all these effects have been observed when administering galantamine in a considerably high therapeutic dose, which is at least 24 mg/day or more. Furthermore, also for galantamine, no synergistic effects on age-related diseases were identified when galantamine is combined with another compound.

Earlier work of the applicant (EP3813882A1) has shown a potentiating and even a synergistic effect on age-related diseases using the biguanide metformin, in combination with the acetylcholinesterase inhibitor galantamine. In particular, this effect was even observed when administering at least one of the compounds, or both compounds, in their subtherapeutic dose.

To overcome therapeutic obstacles such as impaired swallowing and polypharmacy therapy, and also offering some therapeutic benefits such as dose flexibility and combined release patterns, minitablets are a promising patient-friendly drug delivery system (Aleksovski et al. Expert Opinion on Drug Delivery 2014 12, 65). Mini-tablets are tablets typically with a diameter ≤ 3 mm produced on conventional tablet presses equipped with multiple tooling. The production of minitablets is similar to the production of standard tablets but requires excellent powder flow due to the small dies, exact control of process parameters and special caution during tablet press assembly in order to avoid tool damage. Further tablet compositions comprising galantamine are disclosed in WO 2008/062426 A2.

It is therefore an object of the current invention to address problems associated with the production of minitablets, by providing a novel method for the production of galantamine minitablets, the minitablets obtainable by the method, as well the use of galantamine minitablets as a mono-therapeutic, or in combination with other therapeutics, in the treatment of age-related diseases.

### SUMMARY OF THE INVENTION

According to a first aspect, the present invention provides a method for the preparation of an immediate release pharmaceutical composition for oral administration comprising galantamine or a pharmaceutical salt thereof as active pharmaceutical ingredient, said method comprising the following steps: (1) preparing a suspension comprising said active pharmaceutical ingredient and a binder; (2) adding said suspension to a first filler; (3) granulation of the mixture formed in step (2), thereby forming granules; (4) blending the granules of step (3) with a second filler and a disintegrant; (5) blending the granules of step (4) with a lubricant; (6) tableting the granules formed in step (5), thereby forming minitablets.

In particular, the present invention provides a method for the preparation of an immediate release pharmaceutical composition for oral administration comprising galantamine or a pharmaceutical salt thereof as active pharmaceutical ingredient, said method comprising the following steps: (1) preparing a dispersion/solution comprising water and a binder; (2) adding the active ingredient in the form of a dry powder to the dispersion/solution of step (1) to produce a suspension of active pharmaceutical ingredient; (3) adding the suspension of step (2) to a first powdered filler; (4) granulation of the mixture formed in step (3), thereby forming granules; (5) blending the granules of step (4) with a second filler and a disintegrant; (6) blending the granules of step (5) with a lubricant; (7) tableting the granules formed in step (6), thereby forming minitablets.

According to an embodiment of the invention, granulation occurs via fluidized air bed and drying in the same equipment, via high shear granulation and fluid bed air drying, or via high shear granulation and tray drying, preferably via fluidized air bed and drying in the same equipment.

In the different embodiments of the invention, said granules have a mean particle size in the range of 100 micrometers to 400 micrometers.

According to different embodiments of the invention, said minitablets have a mean diameter in the range of 1 to 3 mm, preferably in the range of 1.5 to 2.5 mm, more preferably in the range of 1.8 to 2.2 mm.

In the different embodiments of the invention, said minitablets comprise 15-35 % w/w active pharmaceutical ingredient, 1-5 % w/w binder, 25-50% w/w first filler; 20-40 % w/w second filler, 1-5 % w/w disintegrant and 0.5-5 % w/w lubricant.

In the different embodiments of the invention, said binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof. According to a particular embodiment, said binder is hydroxypropylmethylcellulose.

In the different embodiments of the invention, said first filler is selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol. According to a particular embodiment, said first filler is microcrystalline cellulose.

In the different embodiments of the invention, said second filler is selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol. According to a particular embodiment, said second filler is microcrystalline cellulose.

In the different embodiments of the invention, said disintegrant is selected from the list comprising: crosslinked sodium carboxymethyl cellulose (croscarmellose sodium), crosslinked polyvinylpyrrolidone (crospovidone), sodium starch glycolate, calcium alginate, calcium sodium alginate, calcium carboxymethylcellulose, calcium cellulose glycolate, carmellosum calcium, microcrystalline cellulose, powdered cellulose, chitosan hydrochloride, corn starch, pregelatinized starch and mixtures thereof. According to a particular embodiment, said disintegrant is croscarmellose sodium.

In the different embodiments of the invention, said lubricant is selected from the list comprising: magnesium stearate, sodium stearyl fumarate, calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof. According to a particular embodiment, said lubricant is magnesium stearate.

According to a further aspect, the present invention provides a minitablet obtainable by said method.

According to yet a further aspect, the present invention provides a minitablet comprising 15-35 %w/w galantamine or a pharmaceutical salt thereof as active pharmaceutical ingredient, 1-5 % w/w binder, 25-50% w/w first filler; 20-40 % w/w second filler, 1-5 % w/w disintegrant and 0.5-5 % w/w lubricant.

According to yet a further aspect, the present invention provides a pharmaceutical composition comprising the minitablet according to the different aspects and embodiments of the current invention.

### BRIEF DESCRIPTION OF THE DRAWINGS

**FIG 1****. (A)** shown an illustration of granulation with dry addition, i.e. where the active compound (Gal.HBr) is added to the product container as a dry blend with a filler, and a binder solution is sprayed in the granulation chamber; **(B)** shows an illustration of granulation with wet addition, i.e. where only a filler is added to the product container, and a suspension of the active compound (Gal.HBr) in a binder solution is sprayed in the granulation chamber.
**FIG**. **2** shows the particle size distribution of example 1.
**FIG. 3** shows the dissolution profile of examples 1 in a phosphate buffer at a pH of 6.8.

### DETAILED DESCRIPTION OF THE INVENTION

The present invention will now be further described. In the following paragraphs, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used in the specification and the appended claims, the singular forms "a", "an", and "the" include plural referents unless the context clearly dictates otherwise. By way of example, "a compound" means one compound or more than one compound. The terms described above and others used in the specification are well understood to those in the art.

According to a first aspect, the present invention provides a method for the preparation of an immediate release pharmaceutical composition for oral administration comprising galantamine or a pharmaceutical salt thereof as active pharmaceutical ingredient, said method comprising the following steps: (1) preparing a suspension comprising said active pharmaceutical ingredient and a binder; (2) adding said suspension to a first filler; (3) granulation of the mixture formed in step (2), thereby forming granules; (4) blending the granules of step (3) with a second filler and a disintegrant; (5) blending the granules of step (4) with a lubricant; (6) tableting the granules formed in step (5), thereby forming minitablets.

In particular, the present invention provides a method for the preparation of an immediate release pharmaceutical composition for oral administration comprising galantamine or a pharmaceutical salt thereof as active pharmaceutical ingredient, said method comprising the following steps: (1) preparing a dispersion/solution comprising water and a binder; (2) adding the active ingredient in the form of a dry powder to the dispersion/solution of step (1) to produce a suspension of active pharmaceutical ingredient; (3) adding the suspension of step (2) to a first powdered filler; (4) granulation of the mixture formed in step (3), thereby forming granules; (5) blending the granules of step (4) with a second filler and a disintegrant; (6) blending the granules of step (5) with a lubricant; (7) tableting the granules formed in step (6), thereby forming minitablets.

In a specific embodiment, the powdered active ingredient is added to a dispersion/solution (such as a suspension) of binder in water, and does not require the active ingredient to be dispersed, suspended or dissolved in a solvent such as an alcohol.

In another embodiment, the active ingredient is added to the water/binder solution at a % w/w of about 30 % w/w, such as between 10 and 50 % w/w, in particular between 20 and 50 % w/w. The thus obtained solution is stirred for about 10 - 30 min with a high speed mixer, in particular about 15 min; followed by about 60 - 180 min, in particular about 120 min with a overhead stirrer.

According to an embodiment of the invention, granulation occurs via fluidized air bed and drying in the same equipment, via high shear granulation and fluid bed air drying, or via high shear granulation and tray drying, preferably via fluidized air bed and drying in the same equipment.

In a particular embodiment, the granulation occurs via fluidized air bed using a first powdered filler (typical particle size 20 -400 µm), and does not require the use of beads or tablets (typical particle size 500 - 710 µm). The advantage thereof is that a smaller particles size may be obtained thereby allowing easier further processing step. In a particular embodiment, the particles size of the first powdered filler is between 20 - 400 µm, in particular between 30 - 200 µm, more in particular between 50 - 100 µm.

In a particular embodiment, the first powdered filler is added to the container of the fluid bed equipment and granulation is performed using the suspension of water, binder and active ingredient, wherein the % w/w of filler to suspension is about 50% w/w, in particular between 30 and 70 %w/w, such as between 40 and 60 % w/w.

In the different embodiments of the invention, said granules have a mean particle size in the range of 100 micrometers to 400 micrometers.

According to different embodiments of the invention, said minitablets have a mean diameter in the range of 1 to 3 mm, preferably in the range of 1.5 to 2.5 mm, more preferably in the range of 1.8 to 2.2 mm.

In the different embodiments of the invention, said minitablets comprise 15-35 % w/w active pharmaceutical ingredient, 1-5 % w/w binder, 25-50% w/w first filler; 20-40 % w/w second filler, 1-5 % w/w disintegrant and 0.5-5 % w/w lubricant.

In the different embodiments of the invention, said binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof. According to a particular embodiment, said binder is hydroxypropylmethylcellulose.

In a particular embodiment the suspension containing water and binder has a % w/w of about 4 % w/w, such as between 1 and 10 % w/w, between 2 and 8 % w/w, in particular between 3 and 6 % w/w.

In the different embodiments of the invention, said first filler is selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol. According to a particular embodiment, said first filler is microcrystalline cellulose. In a preferred embodiment, the first filler is a powdered filler.

In the different embodiments of the invention, said second filler is selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol. According to a particular embodiment, said second filler is microcrystalline cellulose. In a preferred embodiment, the second filler is a powdered filler.

**In** a specific embodiment, after the first granulation step, a pre-blend is prepared by blending a second filler, a disintegrant and the granules obtained in the first granulation step, in a ratio of about: 10/1/20 in particular about 9.6/0.9/18.9. After this blending step, a lubricant may be added.

In the different embodiments of the invention, said disintegrant is selected from the list comprising: crosslinked sodium carboxymethyl cellulose (croscarmellose sodium), crosslinked polyvinylpyrrolidone (crospovidone), sodium starch glycolate, calcium alginate, calcium sodium alginate, calcium carboxymethylcellulose, calcium cellulose glycolate, carmellosum calcium, microcrystalline cellulose, powdered cellulose, chitosan hydrochloride, corn starch, pregelatinized starch and mixtures thereof. According to a particular embodiment, said disintegrant is croscarmellose sodium.

In the different embodiments of the invention, said lubricant is selected from the list comprising: magnesium stearate, sodium stearyl fumarate, calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof. According to a particular embodiment, said lubricant is magnesium stearate.

According to a further aspect, the present invention provides a minitablet obtainable by said method.

According to particular embodiments of the invention, said minitablets comprise galantamine or a pharmaceutical salt thereof, hydroxypropylmethylcellulose, microcrystalline cellulose, croscarmellose sodium and magnesium stearate.

According to particular embodiments of the invention, said minitablets comprise 15-35 % w/w galantamine or a pharmaceutical salt thereof, 1-5 % w/w hydroxypropylmethylcellulose, 50-87.5 % w/w microcrystalline cellulose, 1-5 % w/w croscarmellose sodium and 0.5-5 % w/w magnesium stearate.

According to yet a further aspect, the present invention provides a minitablet comprising 15-35 %w/w galantamine or a pharmaceutical salt thereof as active pharmaceutical ingredient, 1-5 % w/w binder, 25-50% w/w first filler; 20-40 % w/w second filler, 1-5 % w/w disintegrant and 0.5-5 % w/w lubricant.

The mini-tablets obtained using the method as defined herein have been shown to have a very fast release profile, wherein about 100% of active ingredient is released within a time frame of only about 10 min, as evident from the examples part.

According to yet a further aspect, the present invention provides a pharmaceutical composition comprising said minitablet according to different aspects and embodiments of the current invention. Such pharmaceutical compositions can be prepared and formulated by methods known in the art and may take a wide variety of forms depending on the form of preparation desired for administration. These pharmaceutical compositions are desirably in unitary dosage form suitable, preferably, for systemic administration such as oral, percutaneous, or parenteral administration.

For example, the minitablets can be formulated along with common excipients, diluents, or carriers, and formed into oral tablets, capsules, sprays, mouth washes, oral liquids (e.g. suspensions, solutions, emulsions), powders, or any other suitable dosage form.

According to an embodiment of the invention, said pharmaceutical composition can be used on its own or as a pharmaceutical combination with another agent used for prevention, stabilization and/or reduction of age-related complaints, degenerative dysfunctioning and/or degenerative complaints. According to an embodiment of the invention, said pharmaceutical composition can be used on its own or as a pharmaceutical combination with another agent for improving a measure of life span and/or health span. According to a particular embodiment, said pharmaceutical composition comprises as another agent a biguanide, and/or an N-oxide, a hydrate, a pharmaceutically acceptable salt or solvate thereof. According to a more particular embodiment, said pharmaceutical composition comprises as another agent metformin.

### EXAMPLES

### Materials

Galantamin.HBr (Gal.HBr) was purchased from Fagron. Croscarmellose sodium (Ac-di-sol SD-711) and microcrystalline cellulose (MCC - Avicel^{®} PH102) were purchased from FMC Health and Nutrition. Magnesium Stearate (Ligamed MF-2-V) was purchased from IMCD Benelux. Colloidal hydrated silica (Syloïd 244FP) was purchased from Grace Davison. Hydroxypropylmethylcellulose (HPMC E5) was purchased from Colorcon.

### Methods

### Bulk and tapped density

The bulk and tapped density of the powders were determined using a Tap Density Tester TD1 (Sotax, Allschwil, Switzerland) equipped with a 25 mL graduated cylinder (readable to 0.5 mL). The tap height and tap speed were set at 3 mm and 250 taps/min. Approximately 25 mL of material was poured into a 25 mL graduated cylinder. The powder weight and exact volume were used to calculate the bulk density (ρB). Subsequently, the sample was subjected to 10, 500, and 1250 taps and the corresponding volumes V10, V500, and V1250 were determined to the nearest graduated unit. In case the difference between V500 and V1250 was less than or equal to 2 mL, V1250 was retained as the tapped volume. When the difference between the volume after 500 taps differed more than 2 mL from the volume obtained after 1250 taps, 1250 extra taps were conducted. The volume reading was then used to determine the tapped density (ρT). Finally, the hausner ratio (HR) and compressibility index (CI) were calculated and used as a measure of powder flowability: $Hausner ratio = \frac{ρT}{ρB}$ $Compressibility index = 100 \times \frac{\left(ρT-ρB\right)}{ρT}$

### Dimensions

Immediately after tableting, the diameter and height of the minitablets (n = 20 per batch) were recorded using a digital caliper (Mahr, Göttingen, Germany).

### Weight

The individual weight of 30 minitablets (of each batch) was recorded using an analytical 5d balance (Sartorius ME235P, Göttingen, Germany).

### Hardness testing

The diametral breaking force of the minitablets (n = 6 per batch) was measured using a pharmaceutical tablet hardness tester (Sotax HT10, Basel, Switzerland).

### Disintegration testing

A Ph. Eur. disintegration apparatus (Sotax DT2, Basel, Switzerland) was used to determine the disintegration time of the minitablets (n = 3 per batch). Considering the small diameter of minitablets, screen opening dimensions were reduced to 1.4 x 1.4 mm. All tests were performed in elixTM water at a temperature of 37 ± 0.5 °C using disks.

### Friability measurements

Minitablet friability was determined by subjecting approximately 6.5 g of minitablets (in accordance with Ph. Eur. Standards) using a friabilator apparatus (Sotax FT2, Basel, Switzerland), set at a speed of 25 rpm for 4 minutes. The percentage weight loss was expressed as the tablet friability.

### Assay testing

Galantamine was quantified by Ultra High-Performance Liquid Chromatography - UPLC (Ph Eur 2.2.29), using UV-absorbance measurement at 230 nm. The evaluation is based on peak-area measurement and external standardization with relative response.

### Particle size distribution

The particle size distribution was performed by sieving the particles with different mesh sizes using a RETSCH type siever, and weighing the different fractions.

### Example 1 - Granulation (wet addition)

### Stage 1 - Suspension

A suspension was prepared in two steps. In a first step a 4 % w/w binder solution was prepared by stirring Methocel E5 Premium LV and purified water for about 45 min. In a second step, Gal.HBr (about 167 g) was added to the binder solution (about 550 g), followed by stirring for about 15 min with a high-speed mixer (SilversonTM L4R, Silverson Machines, Waterside, Chesham, Bucks, England), and finally desaeration for about 120 min using an overhead stirrer.

### Stage 2 - Granulation

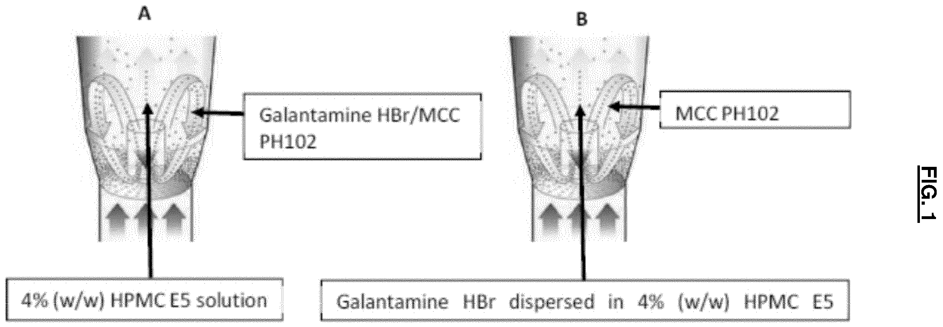

Avicel PH102 (about 333 g) was weighed and introduced into a suitable product container of the fluid bed equipment (Oystar Hüttlin Mycrolab). Granulation was performed with the suspension prepared in the previous stage (about 702 g) according to the parameters listed in Table 1 to obtain white, homogeneous granules, in a yield of 90 %. Figure 1B illustrates the wet addition method for granulation.

**Table 1. Granulation parameters**

| **Granulation parameters** | **Inlet flow rate (m³/h)** | **Inlet temperature (°C)** | **Product temperature (°C)** | **Outlet temperature (°C)** | **Solution flow rate (g/min)** | **Duration (min s)** |
|---|---|---|---|---|---|---|
| Pre-heating | 15-25 | | | | --- | 10-20 min |
| Wetting | 25-35 | | | | 5-15 | 30-60 min |
| Wetting | 35-45 | 50-55 | 25-35 | 25-35 | 5-15 | 45-75 min |
| Drying | 5-15 | | | | --- | 1-10 min |
| Drying | 25-35 | | | | --- | 1-10 min |

Composition data of the obtained suspension and granules is provided in Table 2.

**Table 2. Composition data of Supension & Granules according to Example 1**

| **Suspension** | | | | |
|---|---|---|---|---|
| | **Compound** | | **Mass total batch (g)** | **Relative (%)** |
| | Methocel E5 Premium LV (4 % w/w) | | 548.7 | - |
| | Gal.HBr | | 166.7 | - |

| **Granules** | | | | |
|---|---|---|---|---|
| | **Compound** | | **Mass total batch (g)** | **Relative (%)** |
| | Gal.HBr suspension | | 702.8 | 35.7% |
| | | Methocel E5 Premium LV | 21.6 | 4.2% |
| | | Gal.HBr | 163.8 | 31.6% |
| | Avicel PH102 | | 333.3 | 64.3% |
| | **Subtotal** | | **518.6** | **100.0%** |

Analysis data of the obtained granules is provided in Table 3.

**Table 3. Analysis data of Granules according to Example 1**

| | |
|---|---|
| | |
| Bulk density (g/ml) | 0.336 |
| Tapped density (g/ml) | 0.382 |
| Compressibility index (%) | 12.00 |
| Hausner ratio | 1.14 |
| LOD (%) | 3.26 |
| Assay value (%) | 92.86 |
| | 95.12 |
| | 92.75 |

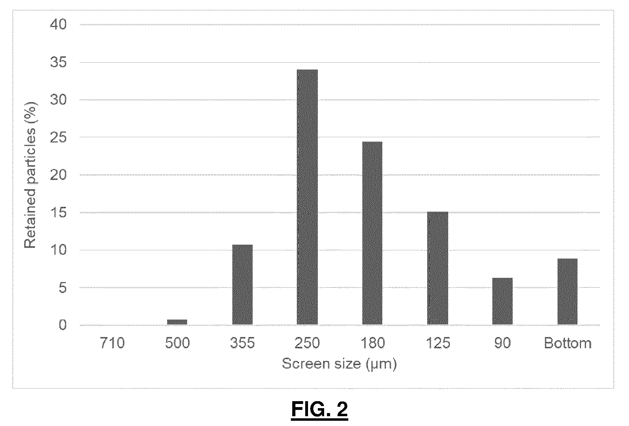

A particle size distribution of the obtained granules is provided in Table 4 and Figure 2.

**Table 4. Particle size distribution of Granules according to Example 1**

| **Screen size (µm)** | **Retained particles (%)** |
|---|---|
| 710 | 0.00 |
| 500 | 0.70 |
| 355 | 10.69 |
| 250 | 33.99 |
| 180 | 24.36 |
| 125 | 15.07 |
| 90 | 6.30 |
| Bottom | 8.89 |
| **Total** | **100.00** |

Observations: The galantamine HBr granules successfully passed powder characterization tests and galantamine assay values were good (i.e. 92.86, 95.12, 92.75%). This was attributed to a larger particle size (distribution) and a decreased loss of fine galantamine HBr powder in the filters of the fluid bed equipment. Hence, assay values of 103.50, 100.96, 103.07% were obtained after assay-purity measurements on material collected from the fluid bed filters.

### Stage 3 - Pre-blend

A pre-blend was prepared by weighing (and sieving on 600 µm) Avicel PH102 (about 9.6 g), Ac-Di-Sol (about 0.9 g) and granules (about 18.9 g) prepared in the previous stage, introducing them in a suitable container and blending for about 10 min using a Turbula blender Type T2F (WAB, Switzerland).

### Stage 4 - Blend

A blend was prepared by weighing, and sieving on 600 µm, magnesium stearate MF2V (about 0,9 g), introducing it in the recipient containing the pre-blend and further blending for about 5 min to obtain a white, homogeneous blend.

Composition data of the obtained blend is provided in Table 5.

**Table 5. Composition data of Pre-Blend & Blend according to Example 1**

| **Pre-Blend** | | | | | |
|---|---|---|---|---|---|
| | **Compound** | | **Mass total batch (g)** | **Mass (mean) per minitablet (mg)** | **Relative (%)** |
| | Gal.HBr granules | | 18.9 | 4.11 | 63.0% |
| | | Methocel E5 Premium LV | 0.79 | 0.17 | 2.6% |
| | | Gal.HBr | 5.97 | 1.30 | 19.9% |
| | | Avicel PH102 | 12.15 | 2.64 | 40.5% |
| | Avicel PH102 | | 9.6 | 2.09 | 32.0% |
| | Ac-Di-Sol | | 0.9 | 0.20 | 3.0% |
| | **Subtotal** | | **29.40** | **6.52** | **98.0%** |

| **Blend** | | | | | |
|---|---|---|---|---|---|
| | **Compound** | | **Mass total batch (g)** | **Mass (mean) per minitablet (mg)** | **Relative (%)** |
| | Magnesium stearate MF2V | | 0.6 | 0.13 | 2.0% |
| **Total Blend** | | | **30** | **6.52** | **100.0%** |

Analysis data of the obtained blend is provided in Table 6.

**Table 6. Analysis data of Blend according to Example 1**

| | |
|---|---|
| | |
| Bulk density (g/ml) | 0.36 |
| Tapped density (g/ml) | 0.47 |
| Compressibility index (%) | 22 |
| Hausner ratio | 1.28 |
| LOD (%) | 4.18 |

### Stage 5 - Tableting

The tableting step was performed on an eccentric tablet press (Korsch XP1, Korsch AG, Berlin, Germany) equipped with 8 D2 mm punches. Tableting parameters: mean compression force: 5.5-6.5 kN.

Analysis data of the obtained tablets is provided in Table 7.

**Table 7. Analysis data of Tablets according to Example 1**

| | |
|---|---|
| | |

| Appearance | White round brightening tablets |
|---|---|
| Friability (n=20) | 0.000 % |
| Hardness (n=6, mean ± SD) | 26 ± 4 N |
| Height (n=20, mean ± SD) | 1.99 ± 0.04 mm |
| Mass (n=20, mean ± SD) | 7.05 ± 0.21 mg |
| Disintegration time (n=3, mean ± SD) | 153 ± 4 sec |

Observations: The resulting minitablets possess a high abrasion resistance. Hence the friability of the minitablets is well below 1.0 (Ph. Eur. Standard for tablets). In addition to a low friability value, the height of the minitablets is similar to the diameter (i.e. 2.0 mm) of the tablets, which results in an aspect ratio of approximately 1. Furthermore, the disintegration time of the minitablets was well below 15 minutes.

### Stage 6 - Dissolution testing

The minitablets were filled into capsules (with a dose strength of eq. 12 mg) and subjected to in vitro dissolution testing using a phosphate buffer at pH 6.8.

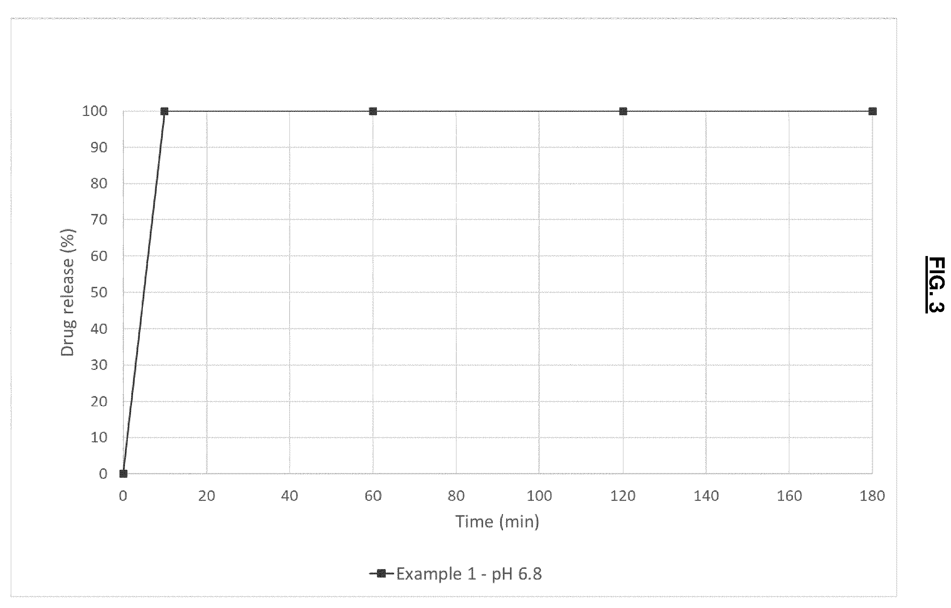

Observations: As shown in Figure 3, immediate release kinetics were observed.

### Example 2 - Granulation (wet addition)

### Stage 1 - Suspension

A suspension was prepared according to Example 1 by adding Gal.HBr (about 67 g) to a 4 % w/w binder solution (about 219.50 g).

### Stage 2 - Granulation

Avicel PH102 (about 133 g) was weighed and introduced into a suitable container. Granulation was performed with the suspension (about 274 g) prepared in the previous stage according to Example 1.

Composition data of the obtained suspension and granules is provided in Table 8.

**Table 8. Composition data of Supension & Granules according to Example 2**

| **Suspension** | | | | |
|---|---|---|---|---|
| | **Compound** | | **Mass total batch (g)** | **Relative (%)** |
| | Methocel E5 Premium LV (4 % w/w) | | 219.5 | - |
| | Gal.HBr | | 66.7 | - |

| **Granules** | | | | |
|---|---|---|---|---|
| | **Compound** | | **Mass total batch (g)** | **Relative (%)** |
| | Gal.HBr suspension | | 273.9 | 35.1% |
| | | Methocel E5 Premium LV | 8.4 | 4.1% |
| | | Gal.HBr | 63.8 | 31.1% |
| | Avicel PH102 | | 133.3 | 64.9% |
| | **Subtotal** | | **205.5** | **100.0%** |

Analysis data of the obtained granules is provided in Table 9.

**Table 9. Analysis data of Granules according to Example 2**

| | |
|---|---|
| | |
| Bulk density (g/ml) | 0.476 |
| Tapped density (g/ml) | 0.496 |
| Compressibility index (%) | 4.00 |
| Hausner ratio | 1.04 |
| LOD (%) | 2.78 |
| Assay value (%) | 78.83 |
| | 79.30 |
| | 80.93 |

Observations: The galantamine HBr granules successfully passed powder characterization tests and galantamine assay values were acceptable (i.e. 78.83, 79.30, 80.93%).

### Comparative example 3 - Direct compression

### Stage 1 - Pre-blend

A pre-blend was prepared by weighing (and sieving on 600 µm) Syloïd 244FP (about 2 g) and Gal.HBr (about 98 g), followed by blending for about 10 min using a Turbula T2A blender.

### Stage 2 - Blend

A blend was prepared by weighing (and sieving on 600 µm) Avicel PH102 (about 25.5 g), Pre-blend (about 3 g), Ac-di-sol (about 0.9 g) and Ligamed MF-2-V (about 0.6 g), subsequently introducing them into a suitable container in the following order: ½ Avicel PH102, Ac-di-sol, Pre-blend, ½ Avicel PH102, blending for about 10 min using a Turbula T2A blender, adding Ligamed MF-2-V to the container and finally blending for about 5 min.

Composition data of the obtained blend is provided in Table 10.

**Table 10. Composition data of Pre-Blend & Blend according to Comparative example 3**

| **Pre-Blend** | | | | |
|---|---|---|---|---|
| | **Compound** | | **Mass total batch (g)** | **Relative (%)** |
| | Gal.HBr | | 98.0 | 98.0% |
| | Syloïd 244FP | | 2.0 | 2.0% |
| | **Subtotal** | | **100.0** | **100.0%** |

| **Blend** | | | | |
|---|---|---|---|---|
| | **Compound** | | **Mass total batch (g)** | **Relative (%)** |
| | Gal.HBr pre-blend | | 3 | 10.0% |
| | | Gal.HBr | 2.94 | 9.8% |
| | | Syloïd 244FP | 0.06 | 0.2% |
| | Avicel PH102 | | 25.5 | 85.0% |
| | Ac-Di-Sol | | 0.9 | 3.0% |
| | Magnesium stearate MF2V | | 0.6 | 2.0% |
| **Total Blend** | | | **30.0** | **100.0%** |

Analysis data of the obtained blend is provided in Table 11.

**Table 11. Analysis data of Blend of Comparative example 3**

| | |
|---|---|
| | |
| Hausner ratio | 1.28 |

### Stage 3 - Tableting

The tableting step was performed on an eccentric tablet press (Korsch XP1, Korsch AG, Berlin, Germany) equipped with 8 D2 mm punches. Tableting parameters: mean compression force: 5.5-6.5 kN.

Observations: Poor powder flow properties (attributed to a Hausner ratio of 1.28), rat holing of the powder bed in the hopper and thus non-homogenous die filling phenomena.

### Comparative example 4 - Direct compression

### Stage 2 - Blend

A blend was prepared by weighing (and sieving on 600 µm) Avicel PH102 (about 26.4 g), Pre-blend of Comparative example 1 (about 1.5 g), Ac-di-sol (about 0.9 g), Ligamed MF-2-V (about 0.6 g) and Syloïd 244FP (about 0.6 g), subsequently introducing them into a suitable container in the following order: ½ Avicel PH102, Ac-di-sol, Preblend, Syloïd 244FP, ½ Avicel PH102, blending for about 10 min using a Turbula T2A blender, adding Ligamed MF-2-V to the container and finally blending for about 5 min.

Composition data of the obtained blend is provided in Table 12.

**Table 12. Composition data of Pre-Blend & Blend according to Comparative example 4**

| **Pre-Blend** | | | | |
|---|---|---|---|---|
| | **Compound** | | **Mass total batch (g)** | **Relative (%)** |
| | Gal.HBr | | 98.0 | 98.0% |
| | Syloïd 244FP | | 2.0 | 2.0% |
| | **Subtotal** | | **100.0** | **100.0%** |

| **Blend** | | | | |
|---|---|---|---|---|
| | **Compound** | | **Mass total batch (g)** | **Relative (%)** |
| | Gal.HBr pre-blend | | 1.5 | 5.0% |
| | | Gal.HBr | 1.47 | 4.9% |
| | | Syloïd 244FP | 0.03 | 0.1% |
| | Avicel PH102 | | 26.4 | 88.0% |
| | Syloïd 244FP | | 0.9 | 3.0% |
| | Ac-Di-Sol | | 0.6 | 2.0% |
| | Magnesium stearate MF2V | | 0.6 | 2.0% |
| **Total Blend** | | | **30.0** | **100.0%** |

Analysis data of the obtained blend is provided in Table 13.

**Table 13. Analysis data of Granules of Comparative example 4**

| | |
|---|---|
| | |
| Compressibility index (%) | 23.69 |
| Hausner ratio | 1.31 |

### Stage 3 - Tableting

The tableting step was performed on an eccentric tablet press (Korsch XP1, Korsch AG, Berlin, Germany) equipped with 8 D2 mm punches. Tableting parameters: mean compression force: 5.5-6.5 kN.

Observations: Passable powder flow properties (attributed to a Hausner ratio of 1.31 and a compressibility index of 23.69) based on bulk/tap density measurements, however rat holing of the powder bed in the hopper and thus non-homogenous die filling phenomena still occurred.

### Comparative example 5 - Granulation (dry addition)

### Stage 2 - Granulation

A dry blend of Gal.HBr (about 67 g) and Avicel PH102 (about 33 g) was weighed and introduced into a suitable product container of the fluid bed equipment (Oystar Hüttlin Mycrolab). Granulation was performed with 4 % w/w binder solution (about 107.7 g) (cf. Example 1), and sieved on 500 µm, to obtain white, homogeneous granules, with a yield of 66%. Figure 1A illustrates the dry addition method for granulation.

Composition data of the obtained suspension and granules is provided in Table 14.

**Table 14. Composition data of Granules according to Comparative example 5**

| **Dry Blend** | | | | |
|---|---|---|---|---|
| | **Compound** | | **Mass total batch (g)** | **Relative (%)** |
| | Gal.HBr | | 66.7 | 66.7% |
| | Syloïd 244FP | | 33.3 | 33.3% |
| | **Subtotal** | | **100.00** | **100.0%** |

| **Granules** | | | | |
|---|---|---|---|---|
| | **Compound** | | **Mass total batch (g)** | **Relative (%)** |
| | Gal.HBr dry blend | | 100 | 95.8% |
| | | Gal.HBr | 66.7 | 63.9% |
| | | Syloïd 244FP | 33.3 | 31.9% |
| | Methocel E5 Premium LV (4 % w/w) | | 107.7 | - |
| | | Methocel E5 Premium LV | 4.4 | 4.2% |
| | **Subtotal** | | **104.4** | **100.0%** |

Analysis data of the obtained granules is provided in Table 15.

**Table 15. Analysis data of Granules of Comparative example 5**

| | |
|---|---|
| | |
| Bulk density (g/ml) | 0.44 |
| Tapped density (g/ml) | 0.478 |
| Compressibility index (%) | 8.00 |
| Hausner ratio | 1.08 |
| LOD (%) | 4.38 |
| Assay value (%) | 56.85 |
| | 57.52 |
| | 55.01 |

Observations: Whereas the galantamine HBr granules successfully passed powder characterization tests, galantamine assay values were low (i.e. 56.85; 57.52 and 55.01%).

### Comparative example 6 - Granulation (dry addition)

### Stage 2 - Granulation

A dry blend of Gal.HBr (about 133 g) and Avicel PH102 (about 67 g) was weighed and introduced into a suitable product container of the fluid bed equipment (Oystar Hüttlin Mycrolab). Granulation was performed with 4 % w/w binder solution (about 219.2 g) (cf. Example 1), and sieved on 500 µm, to obtain white, homogeneous granules, with a yield of 80%.

Composition data of the obtained suspension and granules is provided in Table 16.

**Table 16. Composition data of Granules according to Comparative example 6**

| **Dry Blend** | | | | |
|---|---|---|---|---|
| | **Compound** | | **Mass total batch (g)** | **Relative (%)** |
| | Gal.HBr | | 133.4 | 66.7% |
| | Syloïd 244FP | | 66.6 | 33.3% |
| | **Subtotal** | | **200.0** | **100.0%** |

| **Granules** | | | | |
|---|---|---|---|---|
| | **Compound** | | **Mass total batch (g)** | **Relative (%)** |
| | Gal.HBr dry blend | | 200 | 95.8% |
| | | Gal.HBr | 133.4 | 63.9% |
| | | Syloïd 244FP | 66.6 | 31.9% |
| | Methocel E5 Premium LV (4 % w/w) | | 219.2 | - |
| | | Methocel E5 Premium LV | 8.8 | 4.2% |
| | **Subtotal** | | **208.8** | **100.0%** |

Analysis data of the obtained granules is provided in Table 17.

**Table 17. Analysis data of Granules of Comparative example 6**

| | |
|---|---|
| | |
| Bulk density (g/ml) | 0.492 |
| Tapped density (g/ml) | 0.513 |
| Compressibility index (%) | 4.00 |
| Hausner ratio | 1.04 |
| LOD (%) | 3.06 |
| Assay value (%) | 70.16 |
| | 70.53 |
| | 71.62 |

Observations: Whereas the galantamine HBr granules successfully passed powder characterization tests, galantamine assay values were still low (i.e. 70.16; 70.53 and 71.62%).

## Claims

1. A method for the preparation of an immediate release pharmaceutical composition for oral administration comprising galantamine or a pharmaceutical salt thereof as active pharmaceutical ingredient, said method comprising the following steps:
(1) Preparing a dispersion/solution comprising water and a binder;
(2) Adding the active pharmaceutical ingredient in the form of a dry powder to the dispersion/solution of step (1) to make a suspension of active pharmaceutical ingredient;
(3) Adding the suspension of step (2) to a first powdered filler;
(4) Granulation of the mixture formed in step (3), thereby forming granules;
(5) Blending the granules of step (4) with a second filler and a disintegrant;
(6) Blending the granules of step (5) with a lubricant;
(7) Tableting the granules formed in step (6), thereby forming minitablets.

2. The method according to claim 1, wherein the granules have a mean particle size in the range of 100 micrometers to 400 micrometers.

3. The method according to any one of claims 1 to 2, wherein the minitablets have a mean diameter in the range of 1 to 3 mm, preferably in the range of 1.5 to 2.5 mm, more preferably in the range of 1.8 to 2.2 mm.

4. The method according to any one of claims 1 to 3, wherein the minitablets comprise 15-35 % w/w active pharmaceutical ingredient, 1-5 % w/w binder, 25-50% w/w first filler; 20-40 % w/w second filler, 1-5 % w/w disintegrant and 0.5-5 % w/w lubricant.

5. The method according to any one of claims 1 to 4, wherein the granulation occurs via fluidized air bed and drying in the same equipment, via high shear granulation and fluid bed air drying, or via high shear granulation and tray drying, preferably via fluidized air bed and drying in the same equipment.

6. The method according to any one of claims 1 to 5, wherein the binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof; preferably wherein the binder is hydroxypropylmethylcellulose.

7. The method according to any one of claims 1 to 6, wherein the first filler and the second filler are each independently selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol; preferably wherein the first filler and/or the second filler is microcrystalline cellulose.

8. The method according to any one of claims 1 to 7, wherein the disintegrant is selected from the list comprising: crosslinked sodium carboxymethyl cellulose (croscarmellose sodium), crosslinked polyvinylpyrrolidone (crospovidone), sodium starch glycolate, calcium alginate, calcium sodium alginate, calcium carboxymethylcellulose, calcium cellulose glycolate, carmellosum calcium, microcrystalline cellulose, powdered cellulose, chitosan hydrochloride, corn starch, pregelatinized starch and mixtures thereof; preferably wherein the disintegrant is croscarmellose sodium.

9. The method according to any one of claims 1 to 8, wherein the lubricant is selected from the list comprising: magnesium stearate, sodium stearyl fumarate, calcium stearate, fumaric acid, glyceryl behenate, glyceryl palmitostearate, hydrogenated vegetable oil, magnesium lauryl sulphate, magnesium stearate, sodium lauryl sulphate, sodium stearyl fumarate, starch, stearic acid, talc, zinc stearate and mixtures thereof; preferably wherein the lubricant is magnesium stearate.

10. A minitablet obtainable by the method according to any one of claims 1 to 9.

11. A minitablet comprising 15-35 %w/w galantamine or a pharmaceutical salt thereof as active pharmaceutical ingredient, 1-5 % w/w binder, 25-50% w/w first filler; 20-40 % w/w second filler, 1-5 % w/w disintegrant and 0.5-5 % w/w lubricant.

12. The minitablet according to claim 11, the minitablet having a mean diameter in the range of 1 to 3 mm, preferably in the range of 1.5 to 2.5 mm, more preferably in the range of 1.8 to 2.2 mm.

13. The minitablet according to any one of claims 11 to 12, wherein the binder is selected from the list comprising: hydroxypropylmethylcellulose, acacia gum, alginic acid, carbomer, carboxymethylcellulose calcium, carboxymethycellulose sodium, microcrystalline cellulose, powdered cellulose, ethyl cellulose, gelatine, liquid glucose, guar gum, hydroxyethyl cellulose, hydroxypropyl cellulose, low substituted hydroxypropyl cellulose, hydroxypropylmethyl cellulose (hypromellose), magnesium aluminum silicate, maltodextrin, methylcellulose, polydextrose, polyethylene oxide, povidone, copovidone, sodium alginate, starch paste, pregelatinized starch, sucrose (syrup) and mixtures thereof; preferably wherein the binder is hydroxypropylmethylcellulose.

14. The minitablet according to any one of claims 10 to 13, wherein the first filler and the second filler are each independently selected from the list comprising: microcrystalline cellulose, calcium carbonate, calcium phosphate (dibasic), calcium phosphate (tribasic), calcium sulphate, cellulose, microcrystalline cellulose, microcrystalline silicified cellulose, powdered cellulose, dextrates, dextrose, fructose, lactitol, lactose monohydrate, magnesium carbonate, maltitol, maltodextrin, maltose, mannitol, sodium chloride, sorbitol, starch, pregelatinized starch, sucrose, compressible sugar, xylitol; preferably wherein the first filler and/or the second filler is microcrystalline cellulose.

15. A pharmaceutical composition comprising a minitablet according to any one of claims 10 to 14.

## Patentansprüche

1. Verfahren für die Herstellung einer pharmazeutischen Zusammensetzung mit sofortiger Freisetzung für eine orale Verabreichung, umfassend Galantamin oder ein pharmazeutisches Salz davon als pharmazeutischen Wirkstoff, das Verfahren umfassend die folgenden Schritte:
(1) Herstellen einer Dispersion/Lösung, umfassend Wasser und ein Bindemittel;
(2) Zugeben des pharmazeutischen Wirkstoffs in der Form eines trockenen Pulvers zu der Dispersion/Lösung aus Schritt (1), um eine Suspension des pharmazeutischen Wirkstoffs zu erzeugen;
(3) Zugeben der Suspension aus Schritt (2) zu einem ersten pulverförmigen Füllstoff;
(4) Granulieren der in Schritt (3) ausgebildeten Mischung, wobei dadurch Granulat ausgebildet wird;
(5) Vermischen des Granulats aus Schritt (4) mit einem zweiten Füllstoff und einem Sprengmittel;
(6) Vermischen des Granulats aus Schritt (5) mit einem Gleitmittel;
(7) Tablettieren des in Schritt (6) ausgebildeten Granulats, wobei dadurch Minitabletten ausgebildet werden.

2. Verfahren nach Anspruch 1, wobei die Granulate eine mittlere Partikelgröße in dem Bereich von 100 Mikrometer bis 400 Mikrometer aufweisen.

3. Verfahren nach einem der Ansprüche 1 bis 2, wobei die Minitabletten einen mittleren Durchmesser in dem Bereich von 1 bis 3 mm, vorzugsweise in dem Bereich von 1,5 bis 2,5 mm, mehr bevorzugt in dem Bereich von 1,8 bis 2,2 mm aufweisen.

4. Verfahren nach einem der Ansprüche 1 bis 3, wobei die Minitabletten zu 15-35 Gew.-% den pharmazeutischen Wirkstoff, zu 1-5 Gew.-% das Bindemittel, zu 25-50 Gew.-% den ersten Füllstoff; zu 20-40 Gew.-% den zweiten Füllstoff, zu 1-5 Gew.-% das Sprengmittel und zu 0,5-5 Gew.-% das Gleitmittel umfassen.

5. Verfahren nach einem der Ansprüche 1 bis 4, wobei die Granulierung über eine luftdurchströmte Wirbelschicht und Trocknung in derselben Anlage, über eine Granulierung mit hoher Scherung und Trocknung mit der luftdurchströmten Wirbelschicht oder über eine Granulierung mit hoher Scherung und Hordentrocknung erfolgt, vorzugsweise über die luftdurchströmte Wirbelschicht und Trocknung in derselben Anlage.

6. Verfahren nach einem der Ansprüche 1 bis 5, wobei das Bindemittel aus der Liste ausgewählt ist, umfassend: Hydroxypropylmethylcellulose, Gummiarabicum, Alginsäure, Carbomer, Carboxymethylcellulosecalcium, Carboxymethylcellulosenatrium, mikrokristalline Cellulose, pulverförmige Cellulose, Ethylcellulose, Gelatine, flüssige Glucose, Guargummi, Hydroxyethylcellulose, Hydroxypropylcellulose, niedrig substituierte Hydroxypropylcellulose, Hydroxypropylmethylcellulose (Hypromellose), Magnesiumaluminiumsilicat, Maltodextrin, Methylcellulose, Polydextrose, Polyethylenoxid, Povidon, Copovidon, Natriumalginat, Stärkepaste, prägelatinisierte Stärke, Saccharose (Sirup) und Mischungen davon; wobei das Bindemittel vorzugsweise Hydroxypropylmethylcellulose ist.

7. Verfahren nach einem der Ansprüche 1 bis 6, wobei der erste Füllstoff und der zweite Füllstoff jeweils unabhängig voneinander aus der Liste ausgewählt sind, umfassend: mikrokristalline Cellulose, Calciumcarbonat, Calciumphosphat (dibasisch), Calciumphosphat (tribasisch), Calciumsulfat, Cellulose, mikrokristalline Cellulose, mikrokristalline silifizierte Cellulose, pulverförmige Cellulose, Dextrate, Dextrose, Fructose, Lactit, Lactosemonohydrat, Magnesiumcarbonat, Maltit, Maltodextrin, Maltose, Mannit, Natriumchlorid, Sorbit, Stärke, prägelatinisierte Stärke, Saccharose, komprimierbarer Zucker, Xylit; wobei der erste Füllstoff und/oder der zweite Füllstoff vorzugsweise mikrokristalline Cellulose ist.

8. Verfahren nach einem der Ansprüche 1 bis 7, wobei das Sprengmittel aus der Liste ausgewählt ist, umfassend: vernetzte Natriumcarboxymethylcellulose (Groscarmellosenatrium), vernetztes Polyvinylpyrrolidon (Crospovidon), Natriumstärkeglycolat, Calciumalginat, Calciumnatriumalginat, Calciumcarboxymethylcellulose, Calciumcelluloseglycolat, Carmellosumcalcium, mikrokristalline Cellulose, pulverförmige Cellulose, Chitosanhydrochlorid, Maisstärke, prägelatinisierte Stärkeund Mischungen davon; wobei das Sprengmittel vorzugsweise Croscarmellosenatrium ist.

9. Verfahren nach einem der Ansprüche 1 bis 8, wobei das Gleitmittel aus der Liste ausgewählt ist, umfassend: Magnesiumstearat, Natriumstearylfumarat, Calciumstearat, Fumarsäure, Glycerylbehenat, Glycerylpalmitostearat, hydriertes Pflanzenöl, Magnesiumlaurylsulfat, Natriumlaurylsulfat, Stärke, Stearinsäure, Talkum, Zinkstearat und Mischungen davon; wobei das Gleitmittel vorzugsweise Magnesiumstearat ist.

10. Minitablette, die durch das Verfahren nach einem der Ansprüche 1 bis 9 erhältlich ist.

11. Minitablette, umfassend zu 15-35 Gew.-% das Galantamin oder ein pharmazeutisches Salz davon als pharmazeutischen Wirkstoff, zu 1-5 Gew.-% das Bindemittel, zu 25-50 Gew.-% den ersten Füllstoff; zu 20-40 Gew.-% den zweiten Füllstoff, zu 1-5 Gew.-% das Sprengmittel und zu 0,5-5 Gew.-% das Gleitmittel.

12. Minitablette nach Anspruch 11, wobei die Minitablette einen mittleren Durchmesser in dem Bereich von 1 bis 3 mm, vorzugsweise in dem Bereich von 1,5 bis 2,5 mm, mehr bevorzugt in dem Bereich von 1,8 bis 2,2 mm, aufweist.

13. Minitablette nach einem der Ansprüche 11 bis 12, wobei das Bindemittel aus der Liste ausgewählt ist, umfassend: Hydroxypropylmethylcellulose, Gummiarabicum, Alginsäure, Carbomer, Carboxymethylcellulosecalcium, Carboxymethylcellulosenatrium, mikrokristalline Cellulose, pulverförmige Cellulose, Ethylcellulose, Gelatine, flüssige Glucose, Guargummi, Hydroxyethylcellulose, Hydroxypropylcellulose, niedrig substituierte Hydroxypropylcellulose, Hydroxypropylmethylcellulose (Hypromellose), Magnesiumaluminiumsilicat, Maltodextrin, Methylcellulose, Polydextrose, Polyethylenoxid, Povidon, Copovidon, Natriumalginat, Stärkepaste, prägelatinisierte Stärke, Saccharose (Sirup) und Mischungen davon; wobei das Bindemittel vorzugsweise Hydroxypropylmethylcellulose ist.

14. Minitablette nach einem der Ansprüche 10 bis 13, wobei der erste Füllstoff und der zweite Füllstoff jeweils unabhängig voneinander aus der Liste ausgewählt sind, umfassend: mikrokristalline Cellulose, Calciumcarbonat, Calciumphosphat (dibasisch), Calciumphosphat (tribasisch), Calciumsulfat, Cellulose, mikrokristalline Cellulose, mikrokristalline silifizierte Cellulose, pulverförmige Cellulose, Dextrate, Dextrose, Fructose, Lactit, Lactosemonohydrat, Magnesiumcarbonat, Maltit, Maltodextrin, Maltose, Mannit, Natriumchlorid, Sorbit, Stärke, prägelatinisierte Stärke, Saccharose, komprimierbarer Zucker, Xylit; wobei der erste Füllstoff und/oder der zweite Füllstoff vorzugsweise mikrokristalline Cellulose ist.

15. Pharmazeutische Zusammensetzung, umfassend eine Minitablette nach einem der Ansprüche 10 bis 14.

## Revendications

1. Procédé destiné à la préparation d'une composition pharmaceutique à libération immédiate pour administration orale comprenant de la galantamine ou un sel pharmaceutique de celle-ci en tant qu'ingrédient pharmaceutique actif, ledit procédé comprenant les étapes suivantes :
(1) Préparation d'une dispersion/solution comprenant de l'eau et un liant ;
(2) Ajout de l'ingrédient pharmaceutique actif sous la forme d'une poudre sèche à la dispersion/solution de l'étape (1) pour préparer une suspension d'ingrédient pharmaceutique actif ;
(3) Ajout de la suspension de l'étape (2) à une première charge en poudre ;
(4) Granulation du mélange formé à l'étape (3), permettant ainsi de former des granulés ;
(5) Mélange des granulés de l'étape (4) avec une seconde charge et un désintégrant ;
(6) Mélange des granulés de l'étape (5) avec un lubrifiant ;
(7) Compression des granulés formés à l'étape (6), permettant ainsi de former des minicomprimés.

2. Procédé selon la revendication 1, dans lequel les granulés ont une taille moyenne de particules comprise dans la plage de 100 micromètres à 400 micromètres.

3. Procédé selon l'une quelconque des revendications 1 à 2, dans lequel les minicomprimés ont un diamètre moyen compris dans la plage de 1 à 3 mm, de préférence dans la plage de 1,5 à 2,5 mm, plus préférablement dans la plage de 1,8 à 2,2 mm.

4. Procédé selon l'une quelconque des revendications 1 à 3, dans lequel les minicomprimés comprennent 15 à 35 % p/p d'ingrédient pharmaceutique actif, 1 à 5 % p/p de liant, 25 à 50 % p/p de première charge , 20 à 40 % p/p de seconde charge, 1 à 5 % p/p de désintégrant et 0,5 à 5 % p/p de lubrifiant.

5. Procédé selon l'une quelconque des revendications 1 à 4, dans lequel la granulation se produit par le biais d'un lit d'air fluidisé et d'un séchage dans le même équipement, par le biais d'une granulation à cisaillement élevé et d'un séchage à l'air par lit fluidisé, ou par le biais d'une granulation à cisaillement élevé et d'un séchage en plateau, de préférence par le biais d'un lit d'air fluidisé et d'un séchage dans le même équipement.

6. Procédé selon l'une quelconque des revendications 1 à 5, dans lequel le liant est choisi dans la liste comprenant : hydroxypropylméthylcellulose, gomme arabique, acide alginique, carbomère, carboxyméthylcellulose de calcium, carboxyméthylcellulose de sodium, cellulose microcristalline, cellulose en poudre, éthylcellulose, gélatine, glucose liquide, gomme de guar, hydroxyéthylcellulose, hydroxypropylcellulose, hydroxypropylcellulose à faible substitution, hydroxypropylméthylcellulose (hypromellose), silicate de magnésium et d'aluminium, maltodextrine, méthylcellulose, polydextrose, oxyde de polyéthylène, povidone, copovidone, alginate de sodium, pâte d'amidon, amidon prégélatinisé, saccharose (sirop) et mélanges de ceux-ci ; de préférence dans lequel le liant est l'hydroxypropylméthylcellulose.

7. Procédé selon l'une quelconque des revendications 1 à 6, dans lequel la première charge et la seconde charge sont chacune choisies indépendamment dans la liste comprenant : cellulose microcristalline, carbonate de calcium, phosphate de calcium (dibasique), phosphate de calcium (tribasique), sulfate de calcium, cellulose, cellulose microcristalline, cellulose silicifiée microcristalline, cellulose en poudre, dextrates, dextrose, fructose, lactitol, lactose monohydraté, carbonate de magnésium, maltitol, maltodextrine, maltose, mannitol, chlorure de sodium, sorbitol, amidon, amidon prégélatinisé, saccharose, sucre compressible, xylitol ; de préférence dans lequel la première charge et/ou la seconde charge est la cellulose microcristalline.

8. Procédé selon l'une quelconque des revendications 1 à 7, dans lequel le désintégrant est choisi dans la liste comprenant : carboxyméthylcellulose de sodium réticulée (croscarmellose de sodium), polyvinylpyrrolidone réticulée (crospovidone), glycolate d'amidon de sodium, alginate de calcium, alginate de calcium et de sodium, carboxyméthylcellulose de calcium, glycolate de cellulose et de calcium, carmellose de calcium, cellulose microcristalline, cellulose en poudre, chlorhydrate de chitosane, amidon de maïs, amidon prégélatinisé et mélanges de ceux-ci ; de préférence dans lequel le désintégrant est la croscarmellose de sodium.

9. Procédé selon l'une quelconque des revendications 1 à 8, dans lequel le lubrifiant est choisi dans la liste comprenant : stéarate de magnésium, stéarylfumarate de sodium, stéarate de calcium, acide fumarique, béhénate de glycéryle, palmitostéarate de glycéryle, huile végétale hydrogénée, laurylsulfate de magnésium, laurylsulfate de sodium, amidon, acide stéarique, talc, stéarate de zinc et mélanges de ceux-ci ; de préférence dans lequel le lubrifiant est le stéarate de magnésium.

10. Minicomprimé pouvant être obtenu par le procédé selon l'une quelconque des revendications 1 à 9.

11. Minicomprimé comprenant 15 à 35 % p/p de galantamine ou un sel pharmaceutique de celle-ci en tant qu'ingrédient pharmaceutique actif, 1 à 5 % p/p de liant, 25 à 50 % p/p de première charge ; 20 à 40 % p/p de seconde charge, 1 à 5 % p/p de désintégrant et 0,5 à 5 % p/p de lubrifiant.

12. Minicomprimé selon la revendication 11, le minicomprimé ayant un diamètre moyen compris dans la plage de 1 à 3 mm, de préférence dans la plage de 1,5 à 2,5 mm, plus préférablement dans la plage de 1,8 à 2,2 mm.

13. Minicomprimé selon l'une quelconque des revendications 11 à 12, dans lequel le liant est choisi dans la liste comprenant : hydroxypropylméthylcellulose, gomme arabique, acide alginique, carbomère, carboxyméthylcellulose de calcium, carboxyméthylcellulose de sodium, cellulose microcristalline, cellulose en poudre, éthylcellulose, gélatine, glucose liquide, gomme de guar, hydroxyéthylcellulose, hydroxypropylcellulose, hydroxypropylcellulose à faible substitution, hydroxypropylméthylcellulose (hypromellose), silicate de magnésium et d'aluminium, maltodextrine, méthylcellulose, polydextrose, oxyde de polyéthylène, povidone, copovidone, alginate de sodium, pâte d'amidon, amidon prégélatinisé, saccharose (sirop) et mélanges de ceux-ci ; de préférence dans lequel le liant est l'hydroxypropylméthylcellulose.

14. Minicomprimé selon l'une quelconque des revendications 10 à 13, dans lequel la première charge et la seconde charge sont chacune choisies indépendamment dans la liste comprenant : cellulose microcristalline, carbonate de calcium, phosphate de calcium (dibasique), phosphate de calcium (tribasique), sulfate de calcium, cellulose, cellulose microcristalline, cellulose silicifiée microcristalline, cellulose en poudre, dextrates, dextrose, fructose, lactitol, lactose monohydraté, carbonate de magnésium, maltitol, maltodextrine, maltose, mannitol, chlorure de sodium, sorbitol, amidon, amidon prégélatinisé, saccharose, sucre compressible, xylitol ; de préférence dans lequel la première charge et/ou la seconde charge est la cellulose microcristalline.

15. Composition pharmaceutique comprenant un minicomprimé selon l'une quelconque des revendications 10 à 14.
